# EUROPÄISCHE PATENTSCHRIFT

(11) **EP 0 359 760 B1**
(45) Veröffentlichungstag und Bekanntmachung des Hinweises auf die Patenterteilung: **14.04.1993**
(21) Anmeldenummer: 88903816.2
(22) Anmeldetag: 27.04.1988
(51) Int. Cl.: A61B 5/103

(54) **VORRICHTUNG ZUR BELASTUNGSKONTROLLE VON KÖRPERTEILEN**
DEVICE FOR MONITORING LOADS EXERTED ON PARTS OF THE BODY
DISPOSITIF DE CONTROLE DE CHARGES S'EXERCANT SUR DES PARTIES DU CORPS

(30) Priorität: 29.04.1987 DE 3714218
(43) Veröffentlichungstag der Anmeldung: 28.03.1990
(73) Patentinhaber: HUBERTI, Helmut, D-66740 Saarlouis (DE)
(72) Erfinder: HUBERTI, Helmut, D-66740 Saarlouis (DE)
(74) Vertreter: Knoblauch, Ulrich, Dr.-Ing.
(86) Internationale Anmeldenummer: EP8800354
(87) Internationale Veröffentlichungsnummer: WO8808275

(56) Entgegenhaltungen:
- WO-A-86/04802
- FR-A- 2 273 257
- FR-A- 2 513 508
- MEDICAL & BIOLOGICAL ENGINEERING & COMPUTING, Jahrgang 16, Nr. 5, September 1978; S. MIYAZAKI et al.: "Limb-load alarm device for partial-weight-bearing walking exercise", Seiten 500-505
- MEDICAL & BIOLOGICAL ENGINEERING & COMPUTING, Jahrgang 7, Nr. 2, März 1969; G.C. ROBIN et al.: "Dynamic stress analysis of below-knee drop foot braces; studies on patients with paralysis of the lower limb", Seiten 221-226

## Beschreibung

Die Erfindung bezieht sich auf eine Vorrichtung zur Belastungskontrolle von Körperteilen, wie dem Bewegungsapparat der Beine, mit einer Meßvorrichtung zur Aufnahme eines Belastungsparameters und einem mit deren Meßdaten versorgten, netzunabhängigen, tragbaren Elektronikteil, das eine Eingabevorrichtung zur Eingabe eines Belastungs-Sollbereiches, eine Auswertevorrichtung und eine Anzeigevorrichtung, insbesondere zur Anzeige von Überschreitungen des Sollbereiches, aufweist.

Die therapeutische Teilentlastung von Körperteilen spielt in der Orthopädie und Unfallchirurgie eine große Rolle. Prell- und Stauchungsverletzungen der Gelenke, Knochenbrüche, Band- und Sehnenverletzungen, entzündliche Erkrankungen des Bewegungsapparates sowie Verschleißerkrankungen und Versorgung mit künstlichen Gelenken u.dgl. erforderden zum Teil eine wochen- bzw. monatelange Teilentlastung des betroffenen Körperteils. Hierbei ist das exakte Einhalten eines Teilbelastungsbereiches für eine möglichst rasche Wiederherstellung erforderlich. Hierbei muß eine zu hohe Belastung vermieden, aber eine Mindestbelastung, die den Heilungsvorgang durch mechanische Reize stimuliert, erreicht werden. Der so begrenzte Belastungs-Sollbereich kann während der Behandlungszeit angehoben werden.

Eine Vorrichtung der eingangs beschriebenen Art ist auf dem Markt und in dem Prospektblatt "EDAR Einlage mit Drucksensor und akkustischer Rückmeldung" der Firma Harald Habermann, Orthopädisch-Technische Geräte, Frankfurt/Main beschrieben. Das bekannte batteriebetriebene Gerät besitzt eine Meßvorrichtung, welche die Form einer Einlegesohle mit einem Drucksensor hat. Das Gerät gibt einen tiefen Ton ab, wenn die Meßdaten innerhalb des vorgegebenen Sollbereichs liegen, und einen hohen Ton, wenn der Sollbereich überschritten wird. Der Patient wird daher durch den hohen Ton akkustisch gewarnt, wenn er beim Gehen an Krücken den Bewegungsapparat stärker als vom Arzt vorgeschrieben belastet, und weiß beim tiefen Ton, daß die Belastung richtig ist.

Der Erfindung liegt die Aufgabe zugrunde, eine Vorrichtung zur Belastungskontrolle der eingangs beschriebenen Art dahingehend weiter zu entwickeln, daß das Belastungsverhalten des Patienten besser überprüfbar und insbesondere eine kontinuierliche Langzeit-Belastungskontrolle möglich ist.

Diese Aufgabe wird erfindungsgemäß dadurch gelöst, daß die Auswertevorrichtung einen Mikroprozessor und einen Datenspeicher aufweist, daß dem Datenspeicher Meßdaten von der Meßvorrichtung und weitere der Beschreibung der Belastungsgeschichte dienende Anlaysedaten zuführbar sind, daß dem Datenspeicher zur Einstellung eines Belastungs-Sollbereiches Analysedaten-Sollwerte von der Eingabevorrichtung zuführbar sind, daß das Elektronikteil mit Hilfe des Mikroprozessores die Momentan-Belastung mit dem Sollbereich vergleicht und die so gewonnenen Vergleichsdaten speichert und daß die Anzeigevorrichtung für eine Anzeige gleichzeitig mit der Speicherung ausgelegt ist und/oder daß gespeicherte Daten einer Belastungsgeschichte mittels einer Abrufvorrichtung abrufbar sind.

Bei dieser Vorrichtung stehen zur Auswertung nicht nur die Meßdaten des gemessenen Belastungsparameters zur Verfügung, sondern auch durch Analyse dieser Meßdaten gewonnene Anlaysedaten. Diese haben zum einen den Vorteil, daß sich die Belastungsgeschichte genauer beschreiben und damit die Therapiesteuerung besser gestalten läßt. Beispielsweise erfordert eine längere Überlastung eine andere kompensatorische Entlastung als eine kürzere Überlastung. Zum anderen kann man aus einer Fülle von Meßdaten ein aussagekräftiges Analysedatum gewinnen, so daß die Möglichkeit besteht, die Zahl der zu speichernden und gegebenenfalls mit dem zugehörigen Analysedaten-Sollbereich zu vergleichenden Daten gegenüber der Zahl der Meßdaten zu verringern. Dies vermindert den Aufwand an Speicherraum und an Betriebszeit des Mikroprozessors.

Mit Hilfe der Anzeigevorrichtung kann der Patient sein Belastungsverhalten selbst beobachten, wobei hilfsweise ein akustisches Warnsignal bei Überschreiten des Belastungs-Sollbereichs empfehlenswert ist. Dies ergibt eine erste feed back-Möglichkeit.

Der Datenspeicher nimmt die Meßdaten bzw. einen vom Mikroprozessor ausgewählten Teil hiervon und die hieraus durch den Mikroprozessor aufbereiteten Analysedaten als Momentan- oder Ist-Belastungswerte auf, so daß deren Relation zum zugehörigen Sollbereich errechenbar ist. Er nimmt auch die so gewonnenen Vergleichsdaten auf. Durch den Abruf aller oder eines ausgewählten Teils der gespeicherten Daten, insbesondere der Meß- und Analaysedaten, aber auch der Vergleichsdaten, kann das Belastungsverhalten des Patienten in einer vorgegebenen Periode exakt dokumentiert werden. Dies erlaubt es dem Patienten, jeweils am Abend eine Kontrolle vorzunehmen, um sein Verhalten entsprechend mittelfristig zu verändern. Dies entspricht einer zweiten feed back-Möglichkeit. Besonders günstig es, daß der Arzt jeweils bei der Vorstellung des Patienten dessen Belastungsverhalten nachprüfen und auswerten kann. Hierdurch werden die Heilungschancen für den Patienten verbessert. Es ergibt sich eine optimale Therapiesteuerung und eine exakte forensische Dokumentation. Je nach den Belastungsverhalten des Patienten kann der Belastungs-Sollbereich für die nächste Periode mehr oder weniger erhöht oder überhaupt nicht verändert werden. Dies entspricht einer dritten feed back-Möglichkeit. Insgesamt ergibt sich daher ein abgestuftes feed back-System, das zu einer optimalen Therapiesteuerung beiträgt.

Der gemessene Belastungsparameter ist vorzugsweise eine Belastungskraft. Als Belastunsparameter kommt aber auch eine flächenbezogene Belastungskraft, also eine Druck-, Zug-, Scher- oder Biegebelastung in Betracht. Desweiteren kann der gemessene Belastungsparameter auch eine Bewegungsgröße, insbesondere die Geschwindigkeit oder Beschleunigung, eines Körperteils oder eine andere physikalische Größe sein.

Die Wahl und Anordnung der Meßvorrichtung richtet sich nach dem Anwendungszweck. In vielen Fällen geht es darum, das Bein oder Teile hiervon zu entlasten. In diesem Fall empfiehlt es sich, daß die Meßvorrichtung unterhalb der Fußsohle angeordnet ist, sei es als Einlegesohle, als Therapieschuh oder auch innerhalb oder außerhalb eines Gipsverbandes, beispielsweise in einem Gipsabsatz. Eine solche Meßvorrichtung kann durch zwei im wesentlichen steife Platten gebildet sein, zwischen denen sich drei Sensoren befinden. Mit diesen drei Sensoren ergibt sich eine stabile Auflage und eine hohe Sicherheit dafür, daß die gesamte Kraft von den Sensoren erfaßt wird. Hierbei können die Platten eine Einlegesohle bilden, bei der zwei Sensoren im Ballenbereich und ein Sensor im Fersenbereich angeordnet sind. Die Sensoren können Kraft oder Druck messen und insbesondere mit Dehnungsmeßstreifen ausgestattet sein. Solche Einlegesohlen können preiswert als Wegwerfartikel zum einmaligen Gebrauch konzipiert werden.

Die Meßvorrichtung kann auch anderen Körperteilen zugeordnet werden, beispielsweise anderen Gliedmaßen als dem Fuß, wie der Handinnenfläche bei Athrose-Kranken. Sie kann Gelenken, Knochen, Sehnen und Bändern zugeordnet sein. Dies betrifft auch künstliche Gelenke, Knochen, Sehnen, Bänder und deren Teile. Hierbei kann mindestens eine Meßvorrichtung in den Körper implantiert sein, um die Belastung an Ort und Stelle zu messen. Mindestens eine Meßvorrichtung kann auch zwischen Körper und der Oberfläche von Unterlagen oder Auflagen, die zum Sitzen oder Liegen verwendet werden, angeordnet sein.

Desweiteren kann mindestens eine Meßvorrichtung auch an einer Orthese oder mechanischen Geh-Hilfe angebracht sein, beispielsweise an Kniestützen, Unterarmstockstützen, Achselkrücken oder Gehwagen. Hierbei wird entweder die auf den betreffenden Körperteil wirkende Belastung oder die an ihm vorbei geleitete Belastung gemessen.

Das Gerät ist auch an anderen Stellen einsetzbar, beispielsweise zur Vermeidung von Langzeit-Überlastungsschäden im Hochleistungssport, z.B. bei Marathon-Läufern. Es kann auch als Überlastungsschutz bei vorgeschädigten Gelenken (z.B. Arthrose) benutzt werden, bei denen eine weitere Verschlechterung durch Überlastung verhindert werden muß.

Mit besonderem Vorteil sind die Analysedaten mit Hilfe des Mikroprozessors aus den Meßdaten errechenbar. Diese Aufgabe vermag der Mikroprozessor ohne große Schwierigkeiten zusätzlich zu übernehmen.

Als Analysedaten kommen zahlreiche die Belastung kennzeichnende Daten in Betracht. Hierzu gehören insbesondere die jeweiligen Maxima der Meßdaten in jedem Belastungszyklus. Aus der Fülle von Meßdaten, die der Mikroprozessor im Belastungszyklus abtastet, wird ein einziger Belastungswert als Analysedatum gewonnen.

Besonders vorteilhaft ist es, daß zur Bildung der Analysedaten die Meßdaten in Verbindung mit Zeitwerten auswertbar sind. Dies ergibt eine Reihe von wichtigen zusätzlichen Belastungsangaben.

Insbesondere können als Analysedaten die jeweiligen Belastungszeiten in jeden Belastungszyklus ermittelt werden. Die Belastungszeit ist ein wesentliches Kriterium. Liegt sie außerhalb des Belastungszeit-Sollbereichs, muß der Patient Korrekturen vornehmen. Das Überschreiten des Sollbereichs kann ein akustisches Warnsignal auslösen. Hierbei kann so vorgegangen werden, daß das Elektronikteil mit Hilfe des Mikroprozessors den zeitlichen Abstand zwischen dem Überschreiten und Unterschreiten eines Schwellwerts durch die Meßdaten ermittelt.

Als Analysedaten können auch die Impulsgrößen der Belastungsparameter-Zeit-Kurve in jeden Belastungszyklus verwendet werden. Dies kann insbesondere dadurch geschehen, daß das Elektronikteil mit Hilfe des Mikroprozessors aus den zeitlich aufeinanderfolgenden Meßdaten ein Flächenintegral ermittelt. Auch diese Arbeit kann der Mikroprozessor ohne Schwierigkeiten übernehmen. Die Impulsgrößen kennzeichnen gleichzeitig die mechanische und die zeitliche Belastung.

Vorteilhaft ist es auch, wenn als Analysedatum die Zahl der Belastungszyklen in einer vorgegebenen Zeit verwendet ist. Bei einer Beinbelastung entspricht dies der Schrittzahl. Mit Hilfe des Sollbereichs kann dem Patienten ein bestimmter Schrittzahl-Sollbereich pro Tag vorgegeben werden. Außer den genannten Analysedaten, also Meßwertmaximum, Belastungszeit, Impulsgröße und Zykluszahl können auch hiervon abgeleitete mathematische Funktionen, sei es mit oder ohne Zeitwerte (beispielsweise "pro Tag"), als Analysedaten verwendet werden.

Bei einer bevorzugten Ausführungsform ist dafür gesorgt, daß das Elektronikteil aus den im Datenspeicher gespeicherten Daten, gegebenenfalls unter Verwendung von Zeitwerten, mit Hilfe des Mikroprozessors Statistikwerte errechnet, die in der Anzeigevorrichtung anzeigbar und/oder zwecks späteren Abrufs dem Datenspeicher zugeführt werden. Durch diese Statistikwerte läßt sich die Belastungsgeschichte des Patienten in einer Kurzform darstellen, die dem Patienten oder dem Arzt einen schnellen Überblick und letzerem eine rasche Auswertung ermöglicht. Auch diese zusätzliche Arbeit vermag der Mikroprozessor ohne große Schwierigkeiten zu übernehmen.

Hierbei ist es von großer Hilfe, wenn die in jedem Belastungszyklus ermittelten Analysedaten in Klassen sortiert werden, von denen eine Klasse dem Sollbereich, mindestens eine Klasse dem darüberliegenden Oberbereich und mindestens eine Klasse dem darunterliegenden Unterbereich zugeordnet ist. Durch Einteilung in Klassen kann die Fülle von Daten aus dem Datenspeicher übersichtlich geordnet werden.

Besonders günstig ist es, wenn insgesamt fünf Klassen vorgesehen sind, indem Oberbereich und Unterbereich in je zwei Teilbereiche unterteilt sind. Diese Fünf-Klassen-Einteilung erlaubt gegenüber drei Klassen nicht nur die Beurteilung, ob der Patient den Sollbereich häufig über- oder unterschritten hat, sondern auch, ob das Über- oder Unterschreiten erheblich war oder nicht.

So können als Statistikwerte Prozentzahlen ermittelt werden, die den Zahlen der in die einzelnen Klassen sortierten Analysedaten, bezogen auf die Gesamtzahl der Belastungszyklen in einer vorgegebenen Zeit, entsprechen. Die Prozentzahlen lassen klar erkennen, wie genau sich der Patient an die gegebenen Sollbereichs-Vorschriften gehalten hat. Solche eine Auswertung empfiehlt sich insbesondere hinsichtlich der Meßwertmaxima, kann aber auch für andere Analysedaten verwendet werden.

Bei einer anderen Ausführungsform ist dafür gesorgt, daß die Meßwertmaxima der einzelnen Belastungszyklen in einer vorgegebenen Zeit in Belastungsklassen sortiert werden und als Statistikwerte die zugehörigen Durchschnittswerte der Analysedaten ermittelt werden. Hier werden bestimmte Analysedaten wie Belastungszeit oder Impulsgröße, mit den Meßwertmaxima in Beziehung gebracht, was weitere Beurteilungsmöglichkeiten bietet.

Ferner können mit Vorteil als Statistikwerte die Durchschnittswerte von Analysedaten aus allen Belastungszyklen in einer vorgegebenen Zeit ermittelt werden. Auch dieser Durchschnittswert ist für die Auswertung durch den Arzt von Interesse.

Die Stromversorgung und der Datenspeicher haben vorzugsweise eine Kapazität, also eine Größe, die für einen Betrieb von mehr als einer Woche ausreicht. Die Kapazität sollte so groß sein, daß die Belastungsgeschichte zwischen zwei Vorstellungen beim Arzt in ihren wesentlichen Zügen gespeichert werden kann. Damit Daten nicht verloren gehen, sollte der Datenspeicher auch gegen Stromausfall gesichert sein.

Mit Vorteil weist die Anzeigevorrichtung ein Sichtfenster oder Display auf. Der Patient kann dann optisch den Belastungszustand ablesen, was wesentlich genauer möglich ist als bei einer akustischen Meldung.

Auch die abgerufenen gespeicherten Daten können im Sichtfenster anzeigbar sein. Dies ermöglicht es dem Patienten und dem Arzt, jeweils am Ende eines Tages oder bei der Wiedervorstellung das Belastungsverhalten des Patienten ohne Kenntnis einer Computersprache und ohne besondere Hardware zu ermitteln.

In Weiterbildung der Erfindung kann das Elektronikteil zum Ausdruck der abgerufenen Daten einer Belastungsgeschichte an einen Drucker anschließbar sein. Auch zum Ausdrucken bedarf es keinerlei EDV-Kenntnisse.

Die Eingabevorrichtung und/oder die Abrufvorrichtung können eine Einrichtung zur Aufnehmen eines vorgefertigten Programmträgers aufweisen. Als Programmträger kommt beispielsweise ein Sollbereichs- oder Speicherabruf-EPROM in Betracht.

Stattdessen kann die Eingabevorrichtung und/oder die Abrufvorrichtung auch durch eine Tastatur gebildet sein. Durch Betätigen einiger Tasten werden Werte eingegeben oder der Abrufvorgang eingeleitet.

Desweiteren kann dem Elektronikteil ein tragbarer Meßverstärker vorgeschaltet sein, um auch niedrige Meßdaten im Elektronikteil zu verarbeiten, wenn zu Anfang der Behandlung ein niedriger Soll-Belastungsbereich vorgegeben ist.

Desweiteren ist es günstig, daß der Mikroprozessor eine Kalibrierroutine aufweist, mit deren Hilfe eine Kalibrierung der Meßvorrichtung durchführbar ist. Durch Verwendung des Mikroprozessors, der Eingabevorrichtung und der Anzeigevorrichtung kann man prüfen, ob jeder Sensor den richtigen Meßwert übermittelt und - wenn nicht - diesen Fehler durch einen Korrekturfaktor bei der Meßwert-Auswertung korrigieren.

Zum Stand der Technik auf dem Nachbargebiet der Anzeige des Druckprofils eines belasteten Fußes ist ein Meßsystem bekannt, das ebenfalls Sensor-Einlegesohlen in verschiedenen Schuhgrößen umfaßt, jedoch mit einer großen Anzahl von Sensoren zur Druckverteilungsmessung, zu deren Auswertung ein erheblicher Aufwand für Hardware und Software erforderlich ist. Dieses Meßsystem erfordert EDV-Kenntnisse und ist zur Ausstattung der Praxis eines Orthopäden oder orthopädischen Schuhmachers konzipiert.

Die Erfindung wird nachstehend anhand in der Zeichnung dargestellter bevorzugter Ausführungsbeispiele näher erläutert. Es zeigen:
- Fig. 1: einen Patienten mit angelegte Vorrichtung gemäß der Erfindung,
- Fig. 2: das Elektronikteil in schaubildlicher Darstellung,
- Fig. 3: eine Meßvorrichtung in Form einer Wegwerf-Einlegesohle in Draufsicht,
- Fig. 4: ein Blockschaltbild der erfindungsgemäßen Vorrichtung und
- Fig. 5: ein Belastungs-Zeit-Diagramm.

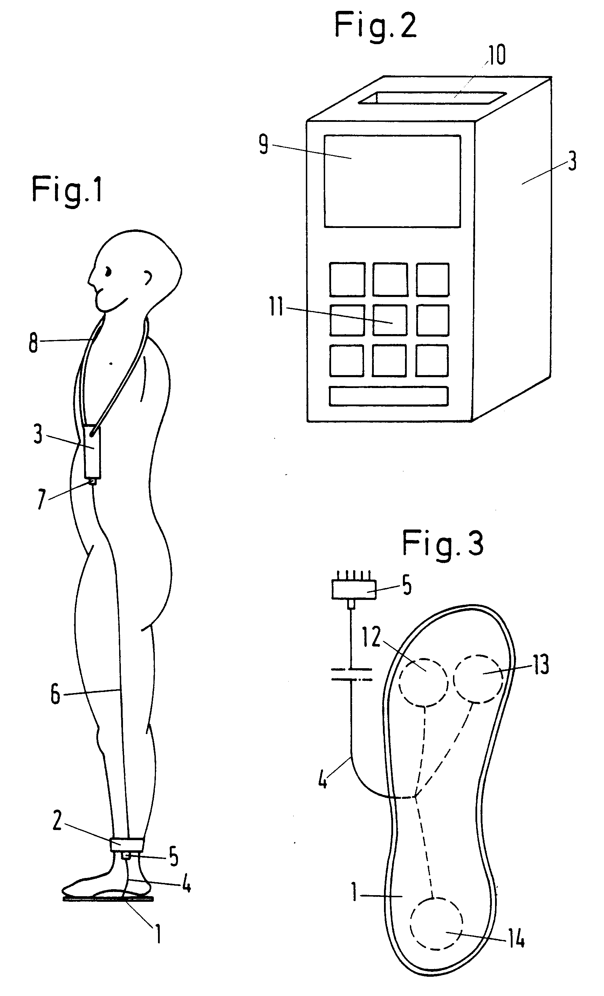

Die Vorrichtung der Fig. 1 bis 3 besteht aus einer Meßvorrichtung in Form einer Einlegesohle 1, einem Meßverstärker 2 sowie einem Elektronikteil 3. Aus der Einlegesohle 1 ist ein Kabel 4 mit einer Steckverbindung 5 herausgeführt, das gerade so lang ist, um die Meßvorrichtung 1 mit dem im Knöchelbereich angeschnallten Meßverstärker 2 zu verbinden. Von diesem geht ein Kabel 6 mit Stecker 7 zu dem Elektronikteil 3, das zweckmäßig mittels einer Tragschlaufe 8 vor der Brust des Patienten getragen wird.

Das in Fig. 2 dargestellte Elektronikteil 3 enthält - wie hier nicht näher dargestellt ist - einen Mikroprozessor, ein Anzeigesystem und einen Datenspeicher. Zum Anzeigesystem gehört außer einer akustischen Anzeigevorrichtung (z.B. ein Piezopiepser) eine optische Anzeigevorrichtung 9 mit an der Vorderwand befindlichem Sichtfenster. Das Elektronikteil 3 besitzt eine Eingabevorrichtung 10, von der ein Schlitz sichtbar ist, durch den ein vorgefertigter Programmträger, beispielsweise ein Sollbereichs-EPROM, einführbar ist. Ferner ist das Elektronikteil 3 mit einer Tastatur 11 versehen, die zur Eingabe oder zum Abruf weiterer Daten vorgesehen ist.

Die in Fig. 3 dargestellte Einlegesohle besteht im wesentlichen aus zwei randseitig miteinander verbundenen Platten, zwischen denen im Ballenbereich zwei Kraftsensoren 12, 13 sowie im Fersenbereich ein Kraftsensor 14, jeweils mit Dehnungsmeßstreifen, fixiert ist. Die Sensoren sind über das Kabel mit dem mehrpoligen Stecker 5 verbunden, über den nicht nur die Meßdaten weitergeleitet, sondern auch die erforderliche Spannung aus dem Elektronikteil 3 oder dem Meßverstärker 2, die mit Batterien versehen sind, zugeführt wird. Hierdurch und wegen der Verwendung einfachster und preiswertester Sensoren ist die Einlegesohle 1 als Wegwerfartikel konzipiert.

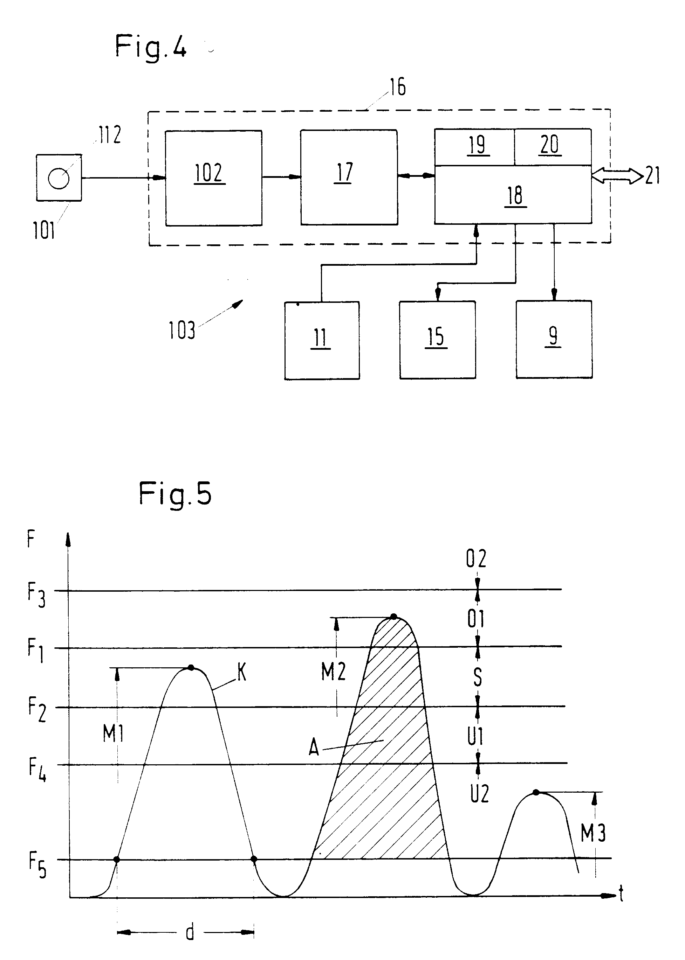

Das Blockschaltbild der Fig. 4 entspricht weitgehend der Darstellung der Fig. 1 bis 3. Der Meßverstärker 102 ist im Elektronikteil 103 und nicht getrennt davon untergebracht, so daß entweder das Elektronikteil am Fußgelenk angebracht werden kann oder ein längeres Kabel 4 benutzt werden muß. Die Meßvorrichtung 101 weist lediglich einen Drucksensor 112 auf. Dieser ist zwischen zwei aufeinanderliegenden Teilen eines künstlichen Gelenks implantiert oder zwischen zwei Teilen einer Gehhilfe angeordnet. Für die übrigen Teile werden die gleichen Bezugszeichen wie zuvor verwendet.

Das Elektronikteil 103 weist außer der als Tastatur ausgebildeten Eingabevorrichtung 11 und der optischen Anzeigevorrichtung 9 noch eine akustische Anzeigevorrichtung 15 und eine Auswertevorrichtung 16 auf. Diese umfaßt den Meßverstärker 102, einen A/D-Wandler 17 und einen Mikroprozessor 18 mit Datenspeicher 19 und Programmspeicher 20. In dem Programmspeicher sind die Programme für alle Arbeitsabläufe, die durch den Mikroprozessor gesteuert werden, gespeichert. An den Mikroprozessor 18 schließt eine E/A-Schnittstelle 21 an. Über diese Schnittstelle kann beispielsweise ein Drucker zum Abrufen von gespeicherten Daten oder eine externe Eingabevorrichtung zur Befehls und Dateneingabe angeschlossen werden.

Fig. 5 zeigt ein Belastungs-Zeit-Diagramm mit einer von den Meßdaten K gebildeten Kurve. Als Beispiel ist die Belastungskraft F, die von der Meßvorrichtung 1 aufgenommen wird, über der Zeit t dargestellt. Durch die fest vorgegebenen Grenzwerte F₁, F₂ ist ein Belastungs-Sollbereich S vorgegeben. Darüber befindet sich ein Oberbereich, der durch den Grenzwert F₃ in einem nahen Oberbereich O1 und fernen Oberbereich O2 unterteilt ist. Darunter befindet sich ein Unterbereich, der durch einen Grenzwert F₄ in einen nahen Unterbereich U1 und einem ferner Unterbereich U2 unterteilt ist. Ein weiterer Schwellwert F₅ ist nahe der Null-Linie vorgesehen. Die genannten Grenzwerte können mittels der Eingabevorrichtung 11 in die Auswertevorrichtung 16 eingegeben werden. Hierbei erleichert es die Eingabe, wenn ein festes Verhältnis existiert, beispielsweise F₂ = 0,8 F₁, F₃ = 1,5 F₁, F₄ = 0,5 F₂ und F₅ = 0,1 F₁. Es braucht dann lediglich F₁ eingegeben zu werden.

Die durch Summierung der Ausgangswerte der Sensoren 12, 13, 14 sich ergebende Kurve der Meßdaten K hat einen kontinuierlichen Verlauf. Sie wird in einem vom Mikroprozessor 18 vorgegebenen Zeittakt abgetastet; die gewonne Meßdatenauswahl wird im A/D-Wandler 17 digitalisiert und dem Datenspeicher 19 zugeführt. Aus diesen Meßdaten werden mit Hilfe des Mikroprozessors 18 weitere Analysedaten berechnet. Die dem Datenspeicher zugeführten Meßdaten brauchen dort nicht dauerhaft gespeichert zu werden. Die Speicherdauer richtet sich danach, wie lange sie für die Auswertung oder Anzeige benötigt werden.

Fig. 5 zeigt drei Möglichkeiten der Berechnung von Analysedaten.
a) Es wird jeweils das Meßwertmaximum ermittelt, so daß sich die einzelnen Maxima M1, M2, M3 usw. als Analysedaten ergeben.
b) Es werden die Zeiten festgestellt, an denen die Meßdaten den Schwellwert F₅ überschreiten bzw. unterschreiten. Die so ermittelten Abstände d können als weitere Analysedaten benutzt werden.
c) Die Fläche unterhalb der Kurve K wird während der Belastungszeit d integriert. Es ergeben sich Impulsgrößen A als weitere Analysedaten.
d) Während einer vorgegebenen Zeit, beispielsweise innerhalb eines Tages werden die Belastungzyklen gezählt. Dies geschieht durch die Feststellung, wie oft der Schwellwert F₅ über- und unterschritten worden ist. Die Zahl der Belastungszyklen stellt ein weiteres Analysedatum dar.

Die Belastungsgeschichte wird daher nicht nur durch die Kurve der Meßwerte K, sondern auch durch die abgeleiteten Analysedaten beschrieben.

Die Auswertung dieser Belastung ist in Fig. 5 im Zusammenhang mit dem Beispiel a) näher erläutert. Das erste Maximum M1 liegt im Sollbereich S, das zweite Maximum M2 im nahen Oberbereich O1 und das dritte Maximum M3 im fernen Unterbereich U2. Im Falle des Maximums M2 wird die akustische Anzeigeeinrichtung 15 wirksam gemacht und ein akustisches Warnsignal abgegeben, weil der Sollbereich S überschritten worden ist.

Die genannten Bereiche S, O1, O2, U1, U2 bilden Belastungsklassen. Für die spätere Auswertung kann es ausreichen, wenn für jedes Maximum festgehalten wird, in welcher Belastungsklasse es liegt. Hierbei ist es nicht erforderlich, daß das Maximum exakt bestimmt wird. Es genügt die Feststellung, welcher Grenzwert F₁ bis F₅ als letzter überschritten worden ist. Und selbst diese Aussage kann noch weiter durch Statistikwerte vereinfacht werden, die angeben, wieviel Maxima innerhalb einer vorgegebenen Periode in die einzelnen Klassen gefallen sind bzw. welcher Prozentsatz der einzelnen Belastungszyklen zu den einzelnen Klassen gehört. Dies ergibt eine Kurzfassung der Belastungsgeschichte einer vorgegebenen Periode.

In ähnlicher Weise können auch die anderen Analysedaten durch Vergleich mit einem Analysedaten-Sollbereich und zugehörigen Ober- und Unterbereichen ausgewertet werden. Hierbei sind auch Verknüpfungen in der Art möglich, daß angegeben wird, welchen Durchschnittswert die betreffenden Analysedaten innerhalb der einzelnen Belastungsklassen haben.

Wenn es um die Zahl der Belastungszyklen pro Tag geht, wird der betreffende Momentan-Belastungswert erst am Ende der Periode erreicht, so daß der Vergleich mit dem Sollbereich erst zu diesem Zeitpunkt erfolgt.

Die von der Meßvorrichtung 1 gelieferten Meßdaten und die daraus abgeleiteten Analysedaten können auf Wunsch gleichzeitig mit der Speicherung im Datenspeicher 19 auch in der optischen Anzeigevorrichtung 9 sichtbar gemacht werden. Auch die jeweiligen Vergleichsdaten können auf diese Weise angezeigt werden, so daß der Patient jederzeit weiß, ob er die Belastung steigern kann oder nicht und in welchem Umfang dies möglich ist.

Nach einer Belastungsperiode wird das Elektronikteil 3 dem Arzt zugeleitet, der gespeicherte Daten einer Belastungsgeschichte mittels einer Abrufvorrichtung, beispielsweise der Tastatur 11, zur Anzeigevorrichtung 9 oder zu einem Drucker abrufen kann. Aufgrund der dokumentierten Belastungsgeschichte kann er die für die nächste Periode günstigste Einstellung der Belastungs-Sollbereiche vornehmen. Insbesondere kann er die Sollbereiche für die oben erwähnten Analysedaten neu so vorgegeben, wie es dem Heilungsverlauf am besten entspricht.

Bei einem Ausführungsbeispiel wurden jeweils durch einen Oberwert und einen Unterwert beschriebene Sollbereiche für das Belastungskraftmaximum, die Zahl der Belastungszyklen pro Tag (Schrittzahl) und die Gesamtenergie pro Tag, dargestellt durch die Summe aller Impulsgrößen, sowie ein nur durch den Oberwert gekennzeichneter Sollbereich für die maximale Belastungsdauer (Schrittdauer) eingegeben. Für die Dauer der Überschreitung der Sollbereiche für die Belastungskraft und für die Schrittdauer ertönt ein akustisches Warnsignal.

Der Patient kann jederzeit per Knopfdruck auf der Tastatur 11 die augenblickliche Belastungskraft, die vom Arzt eingegebenen Belastungs-Sollbereiche (insbesondere für Belastungskraft und Schrittzahl), den Durchschnittswert der Belastungsmaxima vom heutigen Tage und die heutige Schrittzahl auf der optische Anzeigevorrichtung 9 darstellen. Bei einer morgendlichen Überprüfung kann er die Überlastungen vom Vortag, dargestellt durch die Zahl der Über- schreitungen und den Höchstwert (als Abweichung vom Soll in Prozent) sowie die Schrittzahl vom Vortag, dargestellt durch die Schrittanzahl und die Abweichung in Prozent, abrufen.

Der Arzt kann außer den eingegebenen Sollbereichen in einer Kurz-Abfrage die Tageszahl seit der letzten Vorstellung, die durchschnittliche Schrittzahl pro Tag, die durchschnittliche Größe der Belastungskraftmaxima sowie die drei höchsten Einzelbelastungen abrufen. Desweiteren kann noch die maximale und minimale Zahl der Belastungszyklen pro Tag und die maximale und minimale Durchschnittsbelastungskraft pro Tag angegeben werden. Darüberhinaus kann in einer Spezialabfrage noch abgerufen werden, wieviel Prozent der Belastungszyklen ihren Maximalwert M1, M2, M3 in den einzelnen Belastungsklassen hatten, wie groß die Durchschnittsdauer d aller Belastungszyklen und diejenige der Belastungszyklen in den einzelnen Belastungsklassen war, und wie groß der Durchschnittswert der Gesamtenergie (dargestellt durch die Summe der Impulsgrößen) pro Tag im Wiedervorstellungs-Zeitraum sowie der Prozentsatz der in die einzelnen Belastungsklassen fallenden Gesamtenergie war. Diese Daten geben dem Arzt einen guten Überblick über die Belastungsgeschichte.

Insgesamt ist daher festzustellen, daß man aus den Meßdaten Analysedaten gewinnen kann, die der Höhe, der Dauer, der Impulsgröße und der Anzahl der Einzelbelastungen entsprechen sowie als abgeleitete mathematische Funktionen von einem, zwei oder drei dieser Werte und als Funktionen dieser gemessenen und/oder abgeleiteten Werte über der Zeit (z.B. "pro Tag") berechnet werden können, wobei diese Werte dem Datenspeicher 19 sowie der Anzeigevorrichtung 9 zuführbar sind. Für bestimmte Belastungen, wie sie durch Meß- oder Analysedaten beschreibbar sind, gibt es durch die Eingabevorrichtung 10 bzw. 11 vorzugebende Sollwertbereiche. Die Ist-Werte dieser Belastungen (Momentan-Belastung) werden mit Hilfe des Mikroprozessors mit den eingegebenen Sollbereichen verglichen. Auch die Vergleichsdaten werden dem Datenspeicher 19 zugeführt und können in Realzeit auch den Anzeigevorrichtungen 9, 15 zugeführt werden. Statistikwerte werden aus den gespeicherten Ist-Werten, den gespeicherten Vergleichsdaten und weiteren Zeitwerten, z.B. Zahl von Tagen, mit Hilfe des Mikroprozessors 18 errechnet. Auch diese Statistikwerte sind in Realzeit sowohl den Anzeigevorrichtungen 9, 15 als auch dem Datenspeicher 19 zuführbar. Bei einer bestimmten Kombination von Sollbereich, Ist-Werten, Zeitwerten und Statistikwerten werden in der akustischen Anzeigevorrichtung 15 eine oder mehrere akustische Signalarten erzeugt, und in der optischen Anzeigevorrichtung 9 werden bestimmte Daten angezeigt.

Die Größe der Batterie oder des Akkumulators und die Größe des Datenspeichers 19 sind so ausgelegt, daß das Elektronikgerät 103 über eine, zwei oder mehr Wochen eingesetzt werden kann, so daß die gesamte Belastungsgeschichte zwischen zwei aufeinander folgenden Vorstellungen beim Arzt speicherbar ist. Hierbei sorgt die Art des Datenspeichers bzw. eine Zusatzbatterie dafür, daß eine Datensicherung und ein Weiterlaufen der Zeituhr möglich ist.

Zum Kalibrieren der Meßvorrichtung wird so vorgegangen, daß mit Hilfe der Tastatur 11 auf eine Kalibrierroutine geschaltet wird. Dann wird jeder einzelne Kraftsensor 12, 13, 14 mit einer Standardkraft belastet. Dies kann beispielsweise dadurch geschehen, daß der Sensor mit einem kleinflächigen Druckkörper so belastet wird, daß eine darunter liegende Waage einen bestimmten Sollwert anzeigt. Wenn dieser Wert nicht in der Anzeigevorrichtung 9 wiedergegeben wird, muß durch Tastenbetätigung die Anzeige so lange verändert werden, bis der Sollwert dargestellt ist. Dies hat zur Folge, daß auch alle anderen Meßdaten des betreffenden Sensors mit Hilfe eines vom Mikroprozessors berücksichtigten Korrekturgliedes in der richtigen Größe berücksichtigt werden.

In den Ausführungsbeispielen ist gezeigt, daß die Meßvorrichtung 1 über Kabel 4, 6 mit dem Elektronikteil 3 verbunden ist. Stattdessen kann aber auch in der Meßvorrichtung ein Sender und im Elektronikteil ein Empfänger zur drahtlosen Übertragung der Meßdaten integriert sein.

Für den Aufbau der Schaltung werden handelsübliche Bausteine benutzt. Beispielsweise sind die folgenden Bauteile verwendet worden:

| | |
|---|---|
| Kraftsensor 12, 13, 14: | Sensoren mit Dehnungsmeßstreifen Typ 125 GF in der Spezifikation SK-06-125 GF-20 C der Firma Measurement Group |
| A/D-Wandler 17: | MAX 134 der Firma Maxim |
| Mikrocomputer mit Mikroprozessor 18, Datenspeicher 19 und Programmspeicher 20: | DS 5000 der Firma Dallas Semiconductors |

## Patentansprüche

1. Vorrichtung zur Belastungskontrolle von Körperteilen, wie dem Bewegungsapparat der Beine, mit einer Meßvorrichtung zur Aufnahme eines Belastungsparameters und einem mit deren Meßdaten versorgten, netzunabhängigen, tragbaren Elektronikteil, das eine Eingabevorrichtung zur Eingabe eines Belastungs-Sollbereiches, eine Auswertevorrichtung und eine Anzeigevorrichtung, insbesondere zur Anzeige von Überschreitungen des Sollbereiches, aufweist, dadurch gekennzeichnet, daß die Auswertevorrichtung (16) einen Mikroprozessor (18) und einen Datenspeicher (19) aufweist, daß dem Datenspeicher Meßdaten (K) von der Meßvorrichtung (1; 101) und weitere der Beschreibung der Belastungsgeschichte dienende Analysedaten (M1, M2, M3; d; A) zuführbar sind, daß dem Datenspeicher zur Einstellung eines Belastungs-Sollbereiches Analysedaten-Sollwerte (F₁, F₂) von der Eingabevorrichtung (10;11) zuführbar sind, daß das Elektronikteil (3) mit Hilfe des Mikroprozessors die Momentan-Belastung mit dem Sollbereich vergleicht und die so gewonnenen Vergleichsdaten speichert und daß die Anzeigevorrichtung (9, 15) für eine Anzeige gleichzeitig mit der Speicherung ausgelegt ist und/oder daß gespeicherte Daten einer Belastungsgeschichte mittels einer Abrufvorrichtung (11) abrufbar sind.

2. Vorrichtung nach Anspruch 1, dadurch gekennzeichnet, daß die Meßvorrichtung (1; 101) als Kraftsensor für eine Belastungskraft (F) ausgebildet ist und einen dieser Kraft entsprechender Ausgangswert als Belastungsparameter ausgibt.

3. Vorrichtung nach Anspruch 1, dadurch gekennzeichnet, daß die Meßvorrichtung (1; 101) als Drucksensor für eine flächenbezogene Belastungskraft ausgebildet ist und einen dieser flächenbezogenen Belastungskraft entsprechender Ausgangswert als Belastungsparameter ausgibt.

4. Vorrichtung nach Anspruch 1, dadurch gekennzeichnet, daß die Meßvorrichtung (1; 101) als Bewegungsgrößensensor ausgebildet ist und einen Bewegungsgrößen entsprechenden Ausgangswert als Belastungsparameter ausgibt.

5. Vorrichtung nach mindestens einem der Ansprüche 1 bis 4, dadurch gekennzeichnet, daß die Meßvorrichtung (1) als Einlegesohle, Therapieschuh oder Teil eines festen Verbandes ausgebildet ist.

6. Vorrichtung nach Anspruch 5, dadurch gekennzeichnet, daß die Meßvorrichtung (1) durch zwei im wesentlichen steife Platten, zwischen denen sich drei Sensoren (12, 13, 14) befinden, gebildet ist.

7. Vorrichtung nach Anspruch 6, dadurch gekennzeichnet, daß die Platten eine Einlegesohle bilden, bei der zwei Sensoren (12,13) im Ballenbereich und ein Sensor (14) im Fersenbereich angeordnet sind.

8. Vorrichtung nach mindestens einem der Ansprüche 1 bis 4, dadurch gekennzeichnet, daß mindestens eine Meßvorrichtung (101) als in den Körper implantierbares Gelenk-, Knochen-, Sehnen- oder Bänderimplantat oder Teil davon ausgebildet ist.

9. Vorrichtung nach mindestens einem der Ansprüche 1 bis 4, dadurch gekennzeichnet, daß mindestens eine Meßvorrichtung (101) als Teil einer Orthese oder mechanischen Geh-Hilfe ausgebildet ist.

10. Vorrichtung nach mindestens einem der Ansprüche 1 bis 9, dadurch gekennzeichnet, daß das Elektronikteil (3) die Analysedaten (M1, M2, M3; d; A) mit Hilfe des Mikroprozessors (18) aus den Meßdaten (K) errechnet.

11. Vorrichtung nach mindestens einem der Ansprüche 1 bis 10, dadurch gekennzeichnet, daß das Elektronikteil (3) als Analysedaten (M1, M2, M3) die jeweiligen Maxima der Meßdaten in jedem Belastungszyklus ermittelt.

12. Vorrichtung nach mindestens einem der Ansprüche 1 bis 10, dadurch gekennzeichnet, daß das Elektronikteil (3) zur Bildung der Analysedaten die Meßdaten in Verbindung mit Zeitwerten auswertet.

13. Vorrichtung nach Anspruch 12, dadurch gekennzeichnet, daß das Elektronikteil (3) als Analysedaten (d) die jeweiligen Belastungszeiten in jedem Belastungszyklus ermittelt.

14. Vorrichtung nach Anspruch 13, dadurch gekennzeichnet, daß das Elektronikteil (3) mit Hilfe des Mikroprozessors (18) den zeitlichen Abstand (d) zwischen dem Überschreiten und Unterschreiten eines Schwellwerts durch die Meßdaten (K) ermittelt.

15. Vorrichtung nach Anspruch 12, dadurch gekennzeichnet, daß das Elektronikteil (3) als Analysedaten (A) die Impulsgrößen der Belastungsparameter-Zeit-Kurve in jedem Belastungszyklus ermittelt.

16. Vorrichtung nach Anspruch 15, dadurch gekennzeichnet, daß das Elektronikteil (3) mit Hilfe des Mikroprozessors (18) aus den zeitlich aufeinanderfolgenden Meßdaten (K) ein Flächenintegral ermittelt.

17. Vorrichtung nach Anspruch 12, dadurch gekennzeichnet, daß das Elektronikteil (3) als Analysedatum die Zahl der Belastungszyklen in einer vorgegebenen Zeit ermittelt.

18. Vorrichtung nach mindestens einem der Ansprüche 1 bis 17, dadurch gekennzeichnet, daß das Elektronikteil (3) aus den im Datenspeicher (19) gespeicherten Daten mit Hilfe des Mikroprozessors (18) Statistikwerte errechnet, die in der Anzeigevorrichtung (9) anzeigbar und/oder zwecks späteren Abrufs dem Datenspeicher (19) zugeführt werden.

19. Vorrichtung nach Anspruch 18, dadurch gekennzeichnet, daß der Elektronikteil (3) die in jedem Belastungszyklus ermittelten Analysedaten in Klassen sortiert, von denen eine Klasse dem Sollbereich (S), mindestens eine Klasse dem darüberliegenden Oberbereich (O1, O2) und mindestens eine Klasse dem darunterliegenden Unterbereich (U1, U2) zugeordnet sind.

20. Vorrichtung nach Anspruch 18 oder 19, dadurch gekennzeichnet, daß das Elektronikteil (3) als Statistikwerte Prozentzahlen ermittelt, die den Zahlen der in die einzelnen Klassen sortierten Analysedaten, bezogen auf die Gesamtzahl der Belastungszyklen in einer vorgegebenen Zeit, entsprechen.

21. Vorrichtung nach mindestens einem der Ansprüche 18 bis 20, dadurch gekennzeichnet, daß das Elektronikteil (3) die Meßwertmaxima (M1, M2, M3) der einzelnen Belastungszyklen in einer vorgegebenen Zeit in Belastungsklassen sortiert und als Statistikwerte die zugehörigen Durchschnittswerte der Analysedaten (M1, M2, M3; d; A) ermittelt.

22. Vorrichtung nach mindestens einem der Ansprüche 18 bis 21, dadurch gekennzeichnet, daß das Elektronikteil (3) als Statistikwerte die Durchschnittswerte von Analysedaten (M1, M2, M3; d; A) aus allen Belastungszyklen in einer vorgegebenen Zeit ermittelt.

23. Vorrichtung nach mindestens einem der Ansprüche 1 bis 22, dadurch gekennzeichnet, daß die Stromversorgung und der Datenspeicher (19) eine Kapazität haben, die für einen Betrieb von mehr als einer Woche ausreicht.

24. Vorrichtung nach mindestens einem der Ansprüche 1 bis 23, dadurch gekennzeichnet, daß die Anzeigevorrichtung (9) ein Sichtfenster aufweist.

25. Vorrichtung nach Anspruch 24, dadurch gekennzeichnet, daß die abgerufenen gespeicherten Daten im Sichtfenster anzeigbar sind.

26. Vorrichtung nach mindestens einem der Ansprüche 1 bis 25, dadurch gekennzeichnet, daß das Elektronikteil (3; 103) zum Ausdruck der abgerufenen Daten einer Belastungsgeschichte an einen Drucker anschließbar ist.

27. Vorrichtung nach mindestens einem der Ansprüche 1 bis 26, dadurch gekennzeichnet, daß die Eingabevorrichtung und/oder die Abrufvorrichtung eine Einrichtung (10) zum Aufnehmen eines vorgefertigten Programmträgers aufweisen.

28. Vorrichtung nach mindestens einem der Ansprüche 1 bis 27, dadurch gekennzeichnet, daß die Eingabevorrichtung und/oder die Abrufvorrichtung durch eine Tastatur (11) gebildet sind.

29. Vorrichtung nach mindestens einem der Ansprüche 1 bis 28, dadurch gekennzeichnet, daß im Elektronikteil (3) eine Kalibrierroutine gespeichert ist und der Mikroprozessor (18) mit ihrer Hilfe eine Kalibrierung der Meßvorrichtung durchführt.

## Claims

1. Arrangement for checking loads on parts of the body, such as the apparatus of locomotion of the legs, having a measuring arrangement for establishing a load parameter and a network-independent, portable electronics portion which is supplied with the measurement data of the measuring arrangement and has an input arrangement for the input of a desired load range, an evaluating arrangement and a display arrangement, in particular for the display of instances when the desired range is exceeded, characterised in that the evaluating arrangement (16) has a microprocessor (18) and a data store (19), in that it is possible to supply to the data store measurement data (K) from the measuring arrangement (1; 101) and further analysis data (M1, M2, M3; d; A) which is used to describe the load history, in that desired analysis data values (F₁, F₂) can be supplied to the data store from the input arrangement (10; 11) for the purpose of setting a desired load range, in that the electronics portion (3), with the aid of the microprocessor, compares the instantaneous load with the desired range and stores the comparison data thus obtained, and in that the display arrangement (9, 15) is designed to give a display simultaneously with the storage and/or in that stored data of a load history can be retrieved by means of a retrieving arrangement (11).

2. Arrangement according to claim 1, characterised in that the measuring arrangement (1; 101) is formed as a force sensor for a load force (F) and outputs an output value, which corresponds to this force, as a load parameter.

3. Arrangement according to claim 1, characterised in that the measuring arrangement (1; 101) is formed as a pressure sensor for a surface-related load force and outputs an output value, which corresponds to this surface-related load force, as a load parameter.

4. Arrangement according to claim 1, characterised in that the measuring arrangement (1; 101) is formed as a motion magnitude-sensor and outputs an output value, which corresponds to magnitudes of motion, as a load parameter.

5. Arrangement according to at least one of the claims 1 to 4, characterised in that the measuring arrangement (1) is formed as an insole, therapy shoe or portion of a fixed dressing.

6. Arrangement according to claim 5, characterised in that the measuring arrangement (1) is formed by means of two substantially rigid plates, between which are located three sensors (12, 13, 14).

7. Arrangement according to claim 6, characterised in that the plates form an insole in which two sensors (12, 13) are arranged in the region of the ball of the foot and one sensor (14) is arranged in the region of the heel.

8. Arrangement according to at least one of the claims 1 to 4, characterised in that at least one measuring arrangement (101) is formed as a joint, bone, tendon or ligament implant, or a part thereof, which can be implanted into the body.

9. Arrangement according to at least one of the claims 1 to 4, characterised in that at least one measuring arrangement (101) is formed as a portion of an orthotic device or mechanical walking aid.

10. Arrangement according to at least one of the claims 1 to 9, characterised in that the electronics portion (3), with the aid of the microprocessor (18), calculates the analysis data (M1, M2, M3; d; A) from the measurement data (K).

11. Arrangement according to at least one of the claims 1 to 10, characterised in that the electronics portion (3) determines, as analysis data (M1, M2, M3), the respective maxima of the measurement data in each load cycle.

12. Arrangement according to at least one of the claims 1 to 10, characterised in that the electronics portion (3), for the purpose of establishing the analysis data, evaluates the measurement data in conjunction with time values.

13. Arrangement according to claim 12, characterised in that the electronics portion (3) determines, as analysis data (d), the respective load times in each load cycle.

14. Arrangement according to claim 13, characterised in that the electronics portion (3), with the aid of the microprocessor (18), determines the temporal interval (d) between the instance when the measurement data (K) exceeds a threshold value and the instance when it falls short of a threshold value.

15. Arrangement according to claim 12, characterised in that the electronics portion (3) determines, as analysis data (A), the pulse magnitudes of the load parameter time curve in each load cycle.

16. Arrangement according to claim 15, characterised in that the electronics portion (3), with the aid of the microprocessor (18), determines a surface integral from the temporally successive measurement data (K).

17. Arrangement according to claim 12, characterised in that the electronics portion (3) determines, as an analysis datum, the number of the load cycles within a preset time.

18. Arrangement according to at least one of the claims 1 to 17, characterised in that the electronics portion (3), with the aid of the microprocessor (18), calculates from the data stored in the data store (19) statistics values which can be displayed in the display arrangement (9) and/or are supplied to the data store (19) for the purpose of being retrieved later.

19. Arrangement according to claim 18, characterised in that the electronics portion (3) sorts the analysis data, determined in each load cycle, into classes, of which one class is associated with the desired range (S), at least one class is associated with the upper range (O1, O2) lying above the desired range and at least one class is associated with the lower range (U1, U2) lying below the desired range.

20. Arrangement according to claim 18 or 19, characterised in that the electronics portion (3) determines, as statistics values, percentage numbers which correspond to the numbers of the analysis data sorted into the individual classes, relative to the total number of the load cycles within a preset time.

21. Arrangement according to at least one of the claims 18 to 20, characterised in that the electronics portion (3) sorts the measured value maxima (M1, M2, M3) of the individual load cycles within a preset time into load classes and determines the pertinent mean values of the analysis data (M1, M2, M3; d; A) as statistics values.

22. Arrangement according to at least one of the claims 18 to 21, characterised in that the electronics portion (3) determines, as statistics values, the mean values of analysis data (M1, M2, M3; d; A) from all the load cycles within a preset time.

23. Arrangement according to at least one of the claims 1 to 22, characterised in that the current supply and the data store (19) have a capacity which suffices for an operation of more than one week.

24. Arrangement according to at least one of the claims 1 to 23, characterised in that the display arrangement (9) has a see-through vision panel.

25. Arrangement according to claim 24, characterised in that the retrieved, stored data can be displayed in the see-through vision panel.

26. Arrangement according to at least one of the claims 1 to 25, characterised in that the electronics portion (3; 103) can be connected to a printer for the purpose of printing out the retrieved data of a load history.

27. Arrangement according to at least one of the claims 1 to 26, characterised in that the input arrangement and/or the retrieving arrangement have a device (10) for receiving a prefabricated program carrier.

28. Arrangement according to at least one of the claims 1 to 27, characterised in that the input arrangement and/or the retrieving arrangement are formed by means of a keypad (11).

29. Arrangement according to at least one of the claims 1 to 28, characterised in that a calibrating routine is stored in the electronics portion (3) and the microprocessor (18) effects a calibration of the measuring arrangement with the aid of said routine.

## Revendications

1. Dispositif pour le contrôle de charges s'exerçant sur des parties du corps comme par exemple sur l'appareil locomoteur des jambes, avec un dispositif de mesure pour le recueil d'un paramètre de charge et une partie électronique portative, non reliée au secteur, alimentée en données de mesure, partie électronique qui comporte un dispositif d'entrée pour l'entrée d'une plage nominale de charges, un dispositif d'analyse et un dispositif d'affichage, notamment pour l'affichage des dépassements de la plage nominale, caractérisé en ce que le dispositif d'analyse (16) comporte un microprocesseur (18) et une mémoire de données (19), en ce que les données de mesure (K) provenant du dispositif de mesure (1 ; 101) et des mesures d'analyse supplémentaires (M1, M2, M3 ; d ; A) servant à décrire l'historique de charge peuvent être amenées à la mémoire de données, en ce qu'à cette mémoire de données peuvent être amenées, pour la mise au point d'une plage nominale de charges, des données d'analyse - valeur nominale - (F₁, F₂) provenant du dispositif d'entrée (10 ; 11), en ce que la partie électronique (3) compare à l'aide du microprocesseur la charge momentanée avec la plage nominale et mémorise les données comparatives ainsi obtenues et en ce que le dispositif d'affichage (9, 15) est conçu pour un affichage simultanément avec la mise en mémoire et/ou en ce que les données mémorisées d'un historique de charge peuvent être interrogées au moyen d'un dispositif d'interrogation (11).

2. Dispositif selon la revendication 1, caractérisé en ce que le dispositif de mesure (1 ; 101) est conçu sous forme de capteur de force pour un effort de charge ou force (F) et fournit une valeur de sortie en tant que paramètre de charge correspondant à cette force.

3. Dispositif selon la revendication 1, caractérisé en ce que le dispositif de mesure (1 ; 101) est conçu sous forme de capteurs de pression pour un effort de charge rapporté à la surface et émet une valeur de sortie correspondant à cet effort de charge rapporté à la surface en tant que paramètre de charge.

4. Dispositif selon la revendication 1, caractérisé en ce que le dispositif de mesure (1 ; 101) est conçu sous forme de capteurs de grandeur de mouvement et émet une valeur de sortie correspondant à une grandeur de mouvement en tant que paramètre de charge.

5. Dispositif selon au moins l'une des revendications 1 à 4, caractérisé en ce que le dispositif de mesure (1) est conçu sous forme de semelle à insérer, de chaussure thérapeutique ou de partie d'un pansement rigide.

6. Dispositif selon la revendication 5, caractérisé en ce que le dispositif de mesure (1) est conçu par deux plaques essentiellement rigides entre lesquelles sont placés trois capteurs (12, 13, 14).

7. Dispositif selon la revendication 6, caractérisé en ce que les plaques constituent une semelle à insérer dans laquelle sont agencés deux capteurs (12, 13) dans la région des orteils et un capteur (14) dans la région du talon.

8. Dispositif selon au moins l'une des revendications 1 à 4, caractérisé en ce qu'au moins un dispositif de mesure (101) est conçu sous forme d'implant ou partie implantable dans le corps d'une articulation, d'un os, d'un tendon ou d'un ligament.

9. Dispositif selon au moins l'une des revendications 1 à 4, caractérisé en ce qu'au moins un dispositif de mesure (101) est conçu en tant que partie d'une orthèse ou d'un auxiliaire mécanique de la marche.

10. Dispositif selon au moins l'une des revendications 1 à 9, caractérisé en ce que la partie électronique (3) calcule les données d'analyse (M1, M2, M3 ; d ; A) à l'aide du microprocesseur (18) à partir des données de mesure (K).

11. Dispositif selon au moins l'une des revendications 1 à 10, caractérisé en ce que la partie électronique (3) détermine en tant que données d'analyse (M1, M2, M3) les maxima respectifs des données de mesure dans chaque cycle de charge.

12. Dispositif selon au moins l'une des revendications 1 à 10, caractérisé en ce que la partie électronique (3) exploite pour la création des données d'analyse les données de mesure en liaison avec des données de temps.

13. Dispositif selon la revendication 12, caractérisé en ce que la partie électronique (3) détermine en tant que données d'analyse (d) les temps de charge respectifs dans chaque cycle de charge.

14. Dispositif selon la revendication 13, caractérisé en ce que la partie électronique (3) détermine à l'aide du microprocesseur (18) l'intervalle de temps (d) entre les dépassements au-dessus et les dépassements au-dessous d'une valeur de seuil par le biais des données de mesure (K).

15. Dispositif selon la revendication 12, caractérisé en ce que la partie électronique (3) détermine en tant que données d'analyse (A) les grandeurs d'impulsion de la courbe paramètre de charge-temps dans chaque cycle de charge.

16. Dispositif selon la revendication 15, caractérisé en ce que la partie électronique (3) détermine à l'aide du microprocesseur (18) une intégrale de surface à partir des données de mesure (K) successives dans le temps.

17. Dispositif selon la revendication 12, caractérisé en ce que la partie électronique (3) détermine en tant que données d'analyse le nombre de cycles de charge dans un temps prédéterminé.

18. Dispositif selon au moins l'une des revendications 1 à 17, caractérisé en ce que la partie électronique (3) calcule des valeurs statistiques à partir des données stockées dans la mémoire de données (19) à l'aide du microprocesseur (18), valeurs de statistique pouvant être affichées dans le dispositif d'affichage (9) et/ou sont amenées à la mémoire de données (19) pour l'interrogation ultérieure.

19. Dispositif selon la revendication 18, caractérisé en ce que la partie électronique (3) trie par classes les données d'analyse déterminées dans chaque cycle de charge, dont une classe est affectée à la plage nominale (S), au moins une classe à la plage supérieure (O1, O2) et au moins une classe à la plage inférieure (U1, U2).

20. Dispositif selon la revendication 18 ou 19, caractérisé en ce que la partie électronique (3) détermine des pourcents comme valeurs statistiques qui correspondent aux chiffres des données d'analyse triées dans les différentes classes, rapportés au nombre total de cycles de charge dans un temps prédéterminé.

21. Dispositif selon au moins l'une des revendications 18 à 20, caractérisé en ce que la partie électronique (3) trie les maxima de valeurs de mesure (M1, M2, M3) des différents cycles de charge dans un temps prédéterminé en classes de charge et détermine en tant que valeurs de statistique les valeurs moyennes correspondantes des données d'analyse (M1, M2, M3 ; d ; A).

22. Dispositif selon au moins l'une des revendications 18 à 21, caractérisé en ce que la partie électronique (3) détermine en tant que valeurs statistiques les valeurs moyennes des données d'analyse (M1, M2, M3 ; d ; A) à partir de tous les cycles de charge dans un temps prédéterminé.

23. Dispositif selon au moins l'une des revendications 1 à 22, caractérisé en ce que l'alimentation en courant et la mémoire de données (19) ont une capacité suffisante pour un fonctionnement supérieur à une semaine.

24. Dispositif selon au-moins l'une des revendications 1 à 23, caractérisé en ce que le dispositif d'affichage (9) présente une fenêtre de visualisation.

25. Dispositif selon la revendication 24, caractérisé en ce que les données mémorisées interrogées sont affichables dans la fenêtre de visualisation.

26. Dispositif selon au moins l'une des revendications 1 à 25, caractérisé en ce que la partie électronique (3 ; 103) peut être raccordée à une imprimante pour la sortie sur imprimante des données interrogées d'un protocole de charge.

27. Dispositif selon au moins l'une des revendications 1 à 26, caractérisé en ce que le dispositif d'entrée et/ou le dispositif d'interrogation comporte un système (10) pour recevoir un support de programme préfabriqué.

28. Dispositif selon au moins l'une des revendications 1 à 27, caractérisé en ce que le dispositif d'entrée et/ou le dispositif d'interrogation sont constitués par un clavier (11).

29. Dispositif selon au moins l'une des revendications 1 à 28, caractérisé en ce qu'un sous-programme d'étalonnage est mémorisé dans la partie électronique (3) et grâce à celui-ci le microprocesseur (18) effectue un étalonnage du dispositif de mesure.
